# EUROPEAN PATENT SPECIFICATION

(11) **EP 2 450 076 B1**
(45) Date of publication and mention of the grant of the patent: **25.10.2017**
(21) Application number: 10189987.0
(22) Date of filing: 04.11.2010
(51) Int. Cl.: A61M 25/00, A61M 25/01

(54) **Package with catheter**
Packung mit Katheter
Paquet avec cathéter

(43) Date of publication of application: 09.05.2012
(73) Proprietor: Curan Medical B.V., 7007 CJ Doetinchem (NL)
(72) Inventor: van Groningen, David, 6921 RZ, Zelhem (NL); van Velthoven, Ad, 4112 JD, Beusichem (NL)
(74) Representative: Nederlandsch Octrooibureau

(56) References cited:
- EP-A1- 0 677 299
- WO-A2-03/008028
- US-A- 3 229 813
- US-A- 3 967 728
- US-A- 4 811 847
- US-A1- 2001 001 443
- US-A1- 2008 183 181
- US-A1- 2008 260 576
- US-A1- 2009 200 187

## Description

### Field of the invention

The present invention relates to a package with a catheter, e.g. a urethral catheter, which can be used for (intermittent) self-catheterization by a patient.

### Prior art

International patent publication WO00/47494 discloses a storage package with a catheter. The catheter is provided with a coated surface, which exhibits a reduced friction when wetted with a wetting liquid. The wetting liquid is provided in the storage package, and thus contacts the coated surface during the entire shelf live.

British patent application GB-A-2 319 507 discloses a packaged catheter with a lubricant activating liquid. In the package which comprises the catheter, also a container with a liquid (water) is provided. Prior to actual use, the container may be opened such that the liquid can activate the coating on the catheter. Document US 2001/0001443 A1 discloses a urinary catheter having a package, a cap and a chamber containing a lubricant in order to lubricate a catheter prior to use. Document WO 03/008028 A2 discloses a urinary catheter package having a lubricating and gripping device at the distal end of the package.

### Summary of the invention

The present invention seeks to provide an improved package for a (urethral) catheter, suitable for every day use by a patient.

According to the present invention, a package is provided for a catheter as defined in claim 1 and subsequent dependent claims. The catheter is e.g. a urethral catheter, which may be used by the patient or a physician. The present invention allows an equal and easy spread of the lubricating agent when opening the package and taking out the catheter, while having an easy to use and easy to store package. This makes the package according to the present invention suitable for intermittent self-catheterization.

In a further embodiment, the main body comprises a first part for receiving the cap, a second part for holding the gel container and a third part for holding a distal end of the catheter, in which the first part has an inner diameter which is less than an inner diameter of the second part, and the third part has an inner diameter which is less than the inner diameter of the second part. This allows to sufficiently seal off the package allowing it to be carried by a person before actual use.

The third part of the main body is arranged to hold the catheter tube in a space which is shorter than the length of the catheter tube. This variant would allow packaging of longer catheters, e.g. urethral catheters for men.

In an even further embodiment, the gel container comprises locking members extending beyond the inner diameter of the first part. This allows to lock the gel container in place inside the package in a simple and reliable manner.

The gel container comprises an inner sealing member (e.g. in the form of a ring) at the second opening, which in co-operation with the catheter (e.g. the connector part of the catheter) seals off the gel container. In a further variant, the catheter may comprise a closing member (e.g. as part of the connector) for sealing of the second opening of the gel container when positioned in the main body. This sealing member aids in preventing lubricating agent from escaping from the package before actual use of the catheter.

In a further embodiment, the outside dimensions of the package are congruent with the catheter. In other words, a first part of the package (where the connector is located) has an outside diameter smaller than an outside diameter of a second part (where the catheter tube is located). By following the shape of the catheter, a package may be provided with minimal dimensions, making it easy to carry and to store.

In an even further embodiment, the length of the package corresponds to the length of the catheter. This again makes possible a package with minimal dimensions, and allows quick identification of the catheter present in the package.

The lubricating agent comprises a gel in a further embodiment, allowing easy assembly of the package.

In order to make the catheter suitable for application by the major part of patients, in a further embodiment, the catheter is made of a material without softening agent, e.g. PVC with a nelaton tip.

In a further embodiment, the cap is made of a transparent material. This allows to have codes on the catheter (or on the catheter connector) to be visible while the package is still closed off. The codes may be dimension codes, application codes, etc..

The cap is provided with one or more openings in a further embodiment, and optionally with a filter element (such as a paper filter). This allows to sterilize the package and its contents after assembly, by gas sterilization.

In a further aspect, the present invention relates to a method for sterilizing a package as defined in claim 12. This is a very efficient and cost-effective manner of sterilizing the package and its contents in a sufficient manner.

### Short description of drawings

The present invention will be discussed in more detail below, using a number of exemplary embodiments, with reference to the attached drawings, in which
Fig. 1 shows a cross sectional view of a package according to an embodiment of the present invention;
Fig. 2a shows a cross sectional view of an embodiment of a main body of the package of Fig. 1;
Fig. 2b shows a cross sectional view of an embodiment of a catheter of the package of Fig. 1;
Fig. 2c shows a cross sectional view of an embodiment of a gel container of the package of Fig. 1;

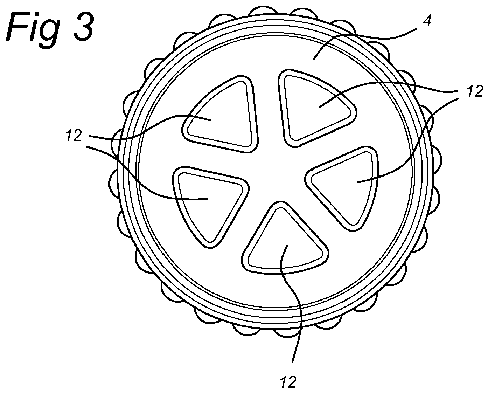

Fig. 3 shows a top view of an embodiment of a cap of the package of Fig. 1.

### Detailed description of exemplary embodiments

The embodiments of the present invention aim to provide an easy to use and easy to carry solution for providing a medical device in the form of a catheter. As an example, the medical device is a urethral catheter allowing to empty the bladder of patient. The present invention embodiments provide for a package in which a urethral catheter 1 can be carried, which is ready for use when taken from the package. The urethral catheter 1 may be used for intermittent self-catheterization.

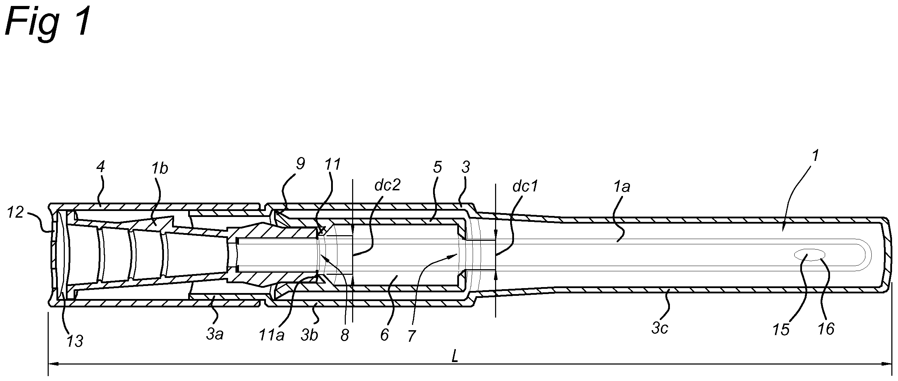

Fig. 1 shows a cross sectional view of an exemplary embodiment of the present invention, showing all elements is assembled state.

A main body 3 is provided which is closed off at a distal end, and which can be closed off at a proximal, open end using a cap 4. The cap 4 can be attached to the main body 3 using various attachment/locking methods, such as a screw thread, a bayonet closure or a clamping arrangement.

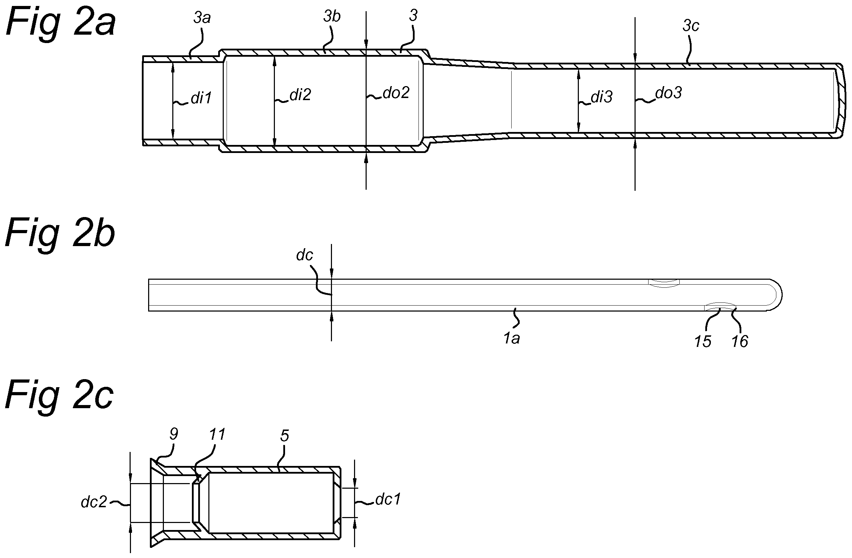

Fig. 2a shows a cross sectional view of the main body 3, which is divided in a first part 3a, second part 3b and third part 3c, which have a first internal diameter di₁, di₂ and di₃, respectively. The first part 3a is arranged for receiving the cap 4, the second part 3b for holding a gel container 5 and the third part 3c for holding the catheter tube 1a of the catheter 1. In the embodiment shown, the first part 3a has an inner diameter di₁ which is less than an inner diameter di₂ of the second part 3b, and the third part 3c has an inner diameter di₃ which is less than the inner diameter di₂ of the second part 3b.

The cap 4 may have a dimension which allows the cap 4 to cover the outside of the first part 3a entirely, thus providing a pen-like appearance for the package. In an embodiment, the cap 4 is made of a transparent material, which allows inspection of the connector 1b of the catheter 1 (which e.g. shows size or other markings on the connector 1b).

As shown in the embodiments of Fig. 1 and Fig. 2a, the outer diameter of the third part 3c may have a transition part from the second part 3b. The outside dimensions of the package are congruent with the general form of the catheter 1, or follows the general dimensions of the catheter 1, i.e. the package 10 is a long, pen shaped package. In more general terms, a first part of the package 10 has an outside diameter smaller than an outside diameter of a second part (i.e. the outer diameter do₃ of the third part 3c of the main body 3 is smaller than the outside diameter do₂ of the second part 3b, as shown in Fig. 2a).

A urethral catheter 1 is shown in the view of Fig. 2b. The urethral catheter 1 is stored in the main body 3, and comprises a distal end in the form of a catheter tube 1a provided with a rounded end and one or more discharge openings 15. The openings 15 are provided with rounded and or polished rims 16, such that the entry of the urethral catheter tube 1a in to the urethral tract is as comfortable as possible for the patient. At a proximal end the catheter 1 is provided with a catheter connector 1b, which may be used to attach the catheter 1 to a collection bag or other collection device. The dimensions of the package 10 (or more specific the internal dimensions of the main body 3 and cap 4) are adapted to allow storage of the entire catheter 1 (which may have varying dimensions). In the embodiment shown, the catheter 1 is about 10-15 cm, which makes this catheter 1 especially suited for use with female patients.

In an embodiment, the length L of the package (see Fig. 1) corresponds to the length of the catheter 1 (i.e. is slightly larger than the catheter 1 so that the package surrounds the entire catheter 1).

The urethral catheter 1 is made of a plastic material without any softening agent in a specific embodiment, in order to prevent possible sensitivity issues with the patient. The material may e.g. be a PVC type of material, and the catheter tube 1a may be provided with a nelaton tip.

Furthermore, a gel container 5 is provided which is positioned in a second part 3b of the main body 3 as shown in the cross sectional view of Fig. 1. In Fig. 2c, the gel container 5 is shown as single element. The gel container 5 is provided with a cavity 6, in which an amount of a gel-like lubricant agent is stored. The gel container 5 comprises a first opening 7 at a distal end, and a second opening 8 at a proximal end, the first opening diameter dc₁ corresponding in general to a diameter dc of the catheter 1, and the second opening dc₂ diameter being larger than the catheter diameter dc. This allows to provide a layer of the gel-like lubricant on the outside surface of the entire catheter 1 when taking the catheter out of the package, making the catheter 1 instantaneously ready for use. The first opening 7 may be provided as a skewed surface in the material of the gel container 5, with a diameter dc₁ smaller than the outer diameter dc of the catheter tube 1b, the flexible material then providing a good sealing of the first opening 7 against the outer wall of the catheter tube 1b.

The lubricating agent comprises a gel-like material in an embodiment of the present invention. In further embodiments, additional components may be included in the lubricating agent, such as compositions having a medicinal function.

The gel container 5 is held in position in the main body 3 by matching he dimensions of the gel container 5 to the inner diameter di₂ of the second part 3b. The gel container 5 is provided with one or more locking members 9, which extend beyond the inner diameter di₁ of the first part 3a. During manufacturing, the gel container 5 can be forced into position in the second part 3b of the main body 3, and the locking members 9 assure a proper fixation of the gel container 5. The locking members are e.g. extensions of the material of the gel container 5, or a ring like extension of flexible material. The gel container 5 is e.g. made of a soft material, such as silicone.

In the embodiment shown in Fig. 1, the gel container 5 comprises an inner sealing member 11 at the second opening 8, which in co-operation with the catheter 1 (e.g. using a properly shaped end part 11a of the connector 1b) seals off the gel container 4. also, the inner sealing member 11 provides a constant thickness film on the outer surface of the catheter tube 1b when the catheter 1 is taken out of the package.

In an alternative embodiment, the catheter 1 comprises a closing member, e.g. as part of the connector 1b, for sealing off the second opening 8 of the gel container 4 when the catheter 1 is positioned in the main body 3.

In a further embodiment, the third part 3c of the main body 3 is arranged to hold the catheter tube 1b of the catheter 1 in a space which is shorter than the length of the catheter tube 1b, e.g. in a folded or coiled up manner. This would also allow to use the present invention package embodiments for longer urethral catheters, e.g. intended for use by male patients.

In order to allow the package and catheter 1 to be used by patients themselves, the catheter 1 is sterilized when assembling the package with catheter 1. In a further aspect of the present invention, the cap 4 of the package is provided with one or more openings 12. These openings 12 are sealed off with a filter element 13 (as shown in the cross sectional view of Fig. 1), e.g. in the form of a paper element. After assembling the main body, gel container, catheter and cap, the internal elements of the package (i.e. the inner lumen of the main body 3 and catheter 1) by introducing (e.g. injecting) a sterilizing gas through the one or more openings in the cap 4.

The present invention embodiments have been described above with reference to a number of exemplary embodiments as shown in the drawings. Modifications and alternative implementations of some parts or elements are possible, and are included in the scope of protection as defined in the appended claims.

## Claims

1. Package for a catheter, comprising
a catheter (1) having a catheter tube (1a) with a distal end and a proximal end, the proximal end being provided with a catheter connector (1b);
a main body (3) for holding the catheter (1), the main body being closed off at a distal end, and a cap (4) for closing off the main body (3) at a proximal, open end;
a gel container (5) positioned inside the main body (3), the gel container (5) being provided with a cavity (6) for holding an amount of gel-like lubricating agent, and comprising a first opening (7) at a distal end, and a second opening (8) at a proximal end, the first opening diameter (dc₁) corresponding to a catheter diameter (dc), and the second opening diameter (dc₂) being larger than the catheter diameter (dc).

2. Package according to claim 1, wherein the main body (3) comprises a first part (3a) for receiving the cap (4), a second part (3b) for holding the gel container (5) and a third part (3c) for holding a distal end of the catheter (1), in which the first part (3a) has an inner diameter (di₁) which is less than an inner diameter (di₂) of the second part (3b), and the third part (3c) has an inner diameter (di₃) which is less than the inner diameter (di₂) of the second part (3b).

3. Package according to claim 2, wherein the third part (3c) of the main body (3) is arranged to hold the catheter tube (1a) in a space which is shorter than the length of the catheter tube (1a).

4. Package according to claim 2 or 3, wherein the gel container (5) comprises locking members (9) extending beyond the inner diameter (di₁) of the first part (3a).

5. Package according to any one of claims 1-4, wherein the gel container (5) comprises an inner sealing member (11) at the second opening (8), which in co-operation with the catheter (1) seals off the gel container (5).

6. Package according to any one of claims 1-5, wherein the outside dimensions of the package are congruent with the catheter (1).

7. Package according to any one of claims 1-6, wherein the length (L) of the package corresponds to the length of the catheter (1).

8. Package according to any one of claims 1-7, wherein the lubricating agent comprises a gel.

9. Package according to any one of claims 1-8, wherein the catheter (1) is made of a material without softening agent.

10. Package according to any one of claims 1-9, wherein the cap (4) is made of a transparent material.

11. Package according to any one of claims 1-10, wherein the cap (4) is provided with one or more openings (12).

12. Method for sterilizing a package according to claim11, comprising assembling the main body (3), gel container (5), catheter (1) and cap (4), and sterilizing internal elements of the package by introducing a sterilizing gas through the one or more openings (12) in the cap (4).

## Patentansprüche

1. Verpackungseinheit für einen Katheter, die Folgendes aufweist:
Einen Katheter (1), der ein Katheterrohr (1a) mit einem distalen Ende und einem proximalen Ende besitzt, wobei das proximale Ende mit einem Katheterverbinder (1b) versehen ist;
einen Hauptkörper (3) zum Halten des Katheters (1), wobei der Hauptkörper an einem distalen Ende geschlossen ist, und eine Abdeckung (4) zum Verschließen des Hauptkörpers (3) an einem proximalen, offenen Ende;
einen Gelbehälter (5), der in dem Hauptkörper (3) positioniert ist, wobei der Gelbehälter (5) mit einem Hohlraum (6) zum Halten einer Menge an gelartigem Gleitmittel versehen ist, und eine erste Öffnung (7) an einem distalen Ende und eine zweite Öffnung (8) an einem proximalen Ende aufweist, wobei der erste Öffnungsdurchmesser (dc₁) einem Katheterdurchmesser (dc) entspricht und der zweite Öffnungsdurchmesser (dc₂) größer als der Katheterdurchmesser (dc) ist.

2. Verpackungseinheit nach Anspruch 1, wobei der Hauptkörper (3) ein erstes Teil (3a) zum Aufnehmen der Abdeckung, ein zweites Teil (3b) zum Halten des Gelbehälters (5) und ein drittes Teil (3c) zum Halten eines distalen Endes des Katheters (1) aufweist, wobei der erste Teil (3a) einen Innendurchmesser (di₁) besitzt, der kleiner ist als der Innendurchmesser (di₂) des zweiten Teils (3b), und der dritte Teil (3c) einen Innendurchmesser (di₃) besitzt, der kleiner ist als der Innendurchmesser (di₂) des zweiten Teils (3b).

3. Verpackungseinheit nach Anspruch 2, wobei der dritte Teil (3c) des Hauptkörpers (3) zum Halten des Katheterrohrs (1a) in einem Raum, der kürzer ist als die Länge des Katheterrohrs (1a), angeordnet ist.

4. Verpackungseinheit nach Anspruch 2 oder 3, wobei der Gelbehälter (5) Verriegelungsglieder (9) aufweist, die sich jenseits des Innendurchmesser (di₁) des ersten Teils (3a) erstrecken.

5. Verpackungseinheit nach einem der Ansprüche 1-4, wobei der Gelbehälter (5) ein inneres Dichtglied (11) an der zweiten Öffnung (8) aufweist, die in Zusammenspiel mit dem Katheter (1) den Gelbehälter (5) abdichtet.

6. Verpackungseinheit nach einem der Ansprüche 1-5, wobei die Außendimensionen der Verpackungseinheit deckungsgleich zu dem Katheter (1) sind.

7. Verpackungseinheit nach einem der Ansprüche 1-6, wobei die Länge (L) der Verpackungseinheit der Länge des Katheters (1) entspricht.

8. Verpackungseinheit nach einem der Ansprüche 1-7, wobei das Gleitmittel ein Gel aufweist.

9. Verpackungseinheit nach einem der Ansprüche 1-8, wobei der Katheter (1) aus einem Material ohne Weichmacher hergestellt ist.

10. Verpackungseinheit nach einem der Ansprüche 1-9, wobei die Abdeckung (4) aus einem transparenten Material hergestellt ist.

11. Verpackungseinheit nach einem der Ansprüche 1-10, wobei die Abdeckung (4) mit einer oder mehr Öffnungen (12) versehen ist.

12. Verfahren zum Sterilisieren einer Verpackungseinheit nach Anspruch 11, das den Zusammenbau des Hauptkörpers (3), Gelbehälters (5), Katheters (1) und der Abdeckung (4) und das Sterilisieren der innenliegenden Elemente der Verpackungseinheit durch Einleiten eines Sterilisationsgases durch die eine oder mehr Öffnungen (12) in der Abdeckung (4) aufweist.

## Revendications

1. Emballage pour cathéter, comprenant
un cathéter (1) comportant un tube de cathéter (1a) pourvu d'une extrémité distale et d'une extrémité proximale, l'extrémité proximale étant pourvue d'un connecteur de cathéter (lb) ;
un corps principal (3) destiné à maintenir le cathéter (1), le corps principal étant fermé à une extrémité distale et un capuchon (4) destiné à fermer le corps principal (3) à une extrémité ouverte proximale ;
un récipient de gel (5) positionné à l'intérieur du corps principal (3), le récipient de gel (5) étant pourvu d'une cavité (6) destinée à contenir une quantité d'agent lubrifiant de type gel et comprenant un premier orifice (7) à une extrémité distale et un second orifice (8) à une extrémité proximale, le premier diamètre d'orifice (dc₁) correspondant au diamètre (dc) du cathéter et le second diamètre d'orifice (dc₂) étant supérieur au diamètre (dc) du cathéter.

2. Emballage selon la revendication 1, dans lequel le corps principal (3) comprend une première partie (3a) destinée à recevoir le capuchon (4), une deuxième partie (3b) destinée à maintenir le récipient de gel (5) et une troisième partie (3c) destinée à maintenir une extrémité distale du cathéter (1), dans laquelle la première partie (3a) a un diamètre intérieur (di₁) inférieur à un diamètre intérieur (di₂) de la deuxième partie (3b) et la troisième partie (3c) a un diamètre intérieur (di₃) inférieur au diamètre intérieur (di₂) de la deuxième partie (3b).

3. Emballage selon la revendication 2, dans lequel la troisième partie (3c) du corps principal (3) est adaptée pour maintenir le tube de cathéter (1a) dans un espace qui est plus court que la longueur du tube de cathéter (1a).

4. Emballage selon la revendication 2 ou 3, dans lequel le récipient de gel (5) comprend des éléments de verrouillage (9) s'étendant au-delà du diamètre intérieur (di₁) de la première partie (3a).

5. Emballage selon l'une quelconque des revendications 1 à 4, dans lequel le récipient de gel (5) comprend un élément d'étanchéité intérieur (11) au niveau du second orifice (8) qui, en coopération avec le cathéter (1), obture le récipient de gel (5).

6. Emballage selon l'une quelconque des revendications 1 à 5, dans lequel les dimensions extérieures de l'emballage sont congruentes au cathéter (1).

7. Emballage selon l'une quelconque des revendications 1 à 6, dans lequel la longueur (L) de l'emballage correspond à la longueur du cathéter (1).

8. Emballage selon l'une quelconque des revendications 1 à 7, dans lequel l'agent lubrifiant comprend un gel.

9. Emballage selon l'une quelconque des revendications 1 à 8, dans lequel le cathéter (1) est constitué d'une matière sans plastifiant.

10. Emballage selon l'une quelconque des revendications 1 à 9, dans lequel le capuchon (4) est constitué d'une mtière transparente.

11. Emballage selon l'une quelconque des revendications 1 à 10, dans lequel le capuchon (4) est pourvu d'un ou plusieurs orifices (12).

12. Procédé de stérilisation d'un emballage selon la revendication 11, comprenant l'assemblage du corps principal (3), du récipient de gel (5), du cathéter (1) et du capuchon (4), et la stérilisation des éléments internes de l'emballage par introduction d'un gaz stérilisant à travers le ou les orifices (12) ménagés dans le capuchon (4).
